# EUROPÄISCHE PATENTSCHRIFT

(11) **EP 0 580 644 B1**
(45) Veröffentlichungstag und Bekanntmachung des Hinweises auf die Patenterteilung: **28.06.1995**
(21) Anmeldenummer: 92907778.2
(22) Anmeldetag: 04.04.1992
(51) Int. Cl.: C07D 211/52, C07D 211/48, C07D 211/22, C07D 211/70, A61K 31/445

(54) **1,3,4-TRISUBSTITUIERTE PIPERIDIN-DERIVATE, IHRE HERSTELLUNG UND VERWENDUNG**
1,3,4-TRISUBSTITUTED PIPERIDINE DERIVATIVES, THEIR PRODUCTION AND USE
DERIVES 1,3,4-TRISUBSTITUES DE LA PIPERIDINE, PREPARATION ET UTILISATION DESDITS DERIVES

(30) Priorität: 16.04.1991 DE 4112353
(43) Veröffentlichungstag der Anmeldung: 02.02.1994
(73) Patentinhaber: BASF Aktiengesellschaft, 67063 Ludwigshafen (DE)
(72) Erfinder: STEINER, Gerd, D-6719 Kirchheim (DE); UNGER, Liliane, D-6700 Ludwigshafen (DE); HOFMANN, Hans, Peter, D-6703 Limburgerhof (DE); TESCHENDORF, Hans-Juergen, D-6724 Dudenhofen (DE); BEHL, Berthold, D-6700 Ludwigshafen (DE); BINDER, Rudolf, D-6520 Worms (DE)
(86) Internationale Anmeldenummer: EP9200765
(87) Internationale Veröffentlichungsnummer: WO9218480

(56) Entgegenhaltungen:
- EP-A- 0 337 167
- FR-A- 2 215 233
- US-A- 3 655 533
- ARZNEIMITTEL FORSCHUNG/DRUG RESEARCH Bd. 34, Nr. 3, 1984, Seiten 233 - 240; 'Synthese, physaikalisch-chemische Eigenschaften und orientierende pharmakologische Untersuchungen von Budipin und verwandten 4,4-Diphenylpiperidinen'
- ARZNEIMITTEL FORSCHUNG/DRUG DESIGN Bd. 18, Nr. 10, 1968, Seiten 1263 - 1269; 'Neue piperidinderivate aus herzwirksamen Aminoketonen'
- CHEMICAL ABSTRACTS, vol. 54, no. 22, 25. November 1960, Columbus, Ohio, US; abstract no. 24714C, 'Mannich reaction. III. Formation of piperidinederivatives'

## Beschreibung

Die Erfindung betrifft 1,3,4-trisubstituierte Piperidin-Derivate, Verfahren zu deren Herstellung und deren Anwendung als Arzneimittel.

Es ist bekannt, daß basisch substituierte Butyrophenon-Derivate Wirkungen als Neuroleptika bzw. Gehirnprotektiva aufweisen (US 4,605,655, EP 410 114). Hierbei scheinen die beobachteten Affinitäten zu σ-Rezeptoren eine besondere Rolle zu spielen.

Es wurde nun gefunden, daß 1,3,4-trisubstituierte Piperidin-Derivate der Formel I 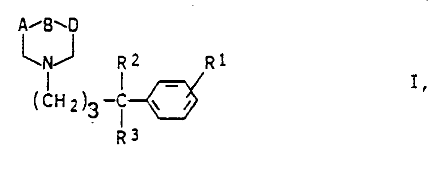
worin
- R¹: ein Wasserstoff-, Fluor-, Chlor- oder Bromatom bedeutet,
- R²: einen Hydroxyrest, einen gegebenenfalls durch ein Fluor-, Chlor- oder Bromatom substituierten Phenylrest darstellt,
- R³: ein Wasserstoffatom bedeutet oder
- R² und R³: zusammen ein Sauerstoffatom darstellen, und
A-B-D die Gruppen 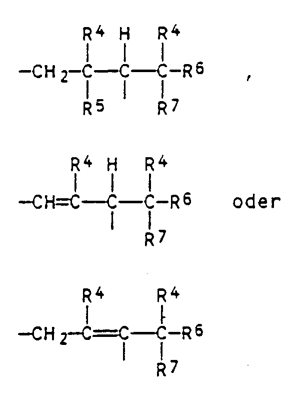
darstellt, worin
- R⁴: einen C₁₋₃-Alkylrest oder eine Phenyl- oder Thienylgruppe darstellt, die durch ein Fluor- oder Chloratom substituiert sein können,
- R⁵: ein Wasserstoffatom oder eine Hydroxygruppen darstellt,
- R⁶: ein Wasserstoffatom,
- R⁷: eine Hydroxygruppe ist oder
- R⁶ und R⁷: zusammen ein Sauerstoffatom bedeuten
und deren Salze mit physiologisch verträglichen Säuren wertvolle pharmakologische Eigenschaften besitzen.

In der Formel I haben die Substituenten R¹ bis R⁷ sowie n vorzugsweise folgende Bedeutungen:
- R¹:: Wasserstoff, Fluor, Chlor
- R²:: Hydroxy, p-Fluorphenyl
- R³:: Wasserstoff oder zusammen mit R² Sauerstoff
- R⁴:: Methyl, Ethyl, Phenyl, p-Fluorphenyl, 2-Thienyl
- R⁵:: Wasserstoff, Hydroxy
- R⁶:: Wasserstoff
- R⁷:: Hydroxy oder mit R⁶ Sauerstoff
Die folgenden Verbindungen sind als besonders bevorzugt zu nennen:
1-(4-Fluorphenyl)-4-[trans-(3-phenyl-hydroxymethyl-4-phenyl)-4-hydroxy-piperidin-1-yl]-butan-1-on, 1-(4-Fluorphenyl)-4-[trans-(3-phenyl-hydroxymethyl-4-phenyl)-4-hydroxy-piperidin-1-yl]-butan-1-ol,
1-(4-Fluorphenyl)-4-(3-benzoyl-4-phenyl-Δ⁴-dehydro-piperidin-1-y l)-butan-1-on, 1-(4-Fluorphenyl)-4-(3-benzoyl-4-phenyl-Δ³-dehydro-piperidin-1-yl)-butan-1-on, 1-(Bis-4-fluorphenyl)-4-[trans-(3-phenyl-hydroxymethyl-4-phenyl)-4-hydroxy-piperidin-1-yl]-butan, 1-(4-Fluorphenyl)-4-[trans-(3-p-fluorphenyl-hydroxymethyl-4-p-fluorphenyl-4-hydroxypiperidin-1-yl]-butan-1-on, 1-(4-Fluorphenyl)-4-[trans-(3-p-fluorphenyl-hydroxymethyl-4-p-fluorphenyl-4-hydroxypiperidin-1-yl]-butan-1-ol, 1-(4-Fluorphenyl)-4-[trans-(3-carbonylmethyl-4-methyl)-4-hydroxy-piperidin-1-yl]-butan-1-on, 1-(4-Fluorphenyl)-4-[trans-(3-carbonylmethyl-4-methyl)-4-hydroxy-piperidin-1-yl]-butan-1-ol, 1-(4-Fluorphenyl)-4-(3-carbonylmethyl-4-methyl-Δ³-dehydropiperidin-1-yl)-butan-1-on, 1-(4-Fluorphenyl)-4-(3-carbonylmethyl-4-methyl-Δ³-dehydropiperidin-1-yl)-butan-1-ol,
Die erfindungsgemäßen Verbindungen der Formel I lassen sich herstellen, indem man eine Verbindung der Formel II 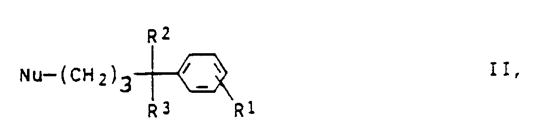
in der R¹, R² und R³ die angegebenen Bedeutungen haben und Nu eine nukleofuge Abgangsgruppe darstellt, mit einem 3,4-disubstituierten Piperidin-Derivat der Formel III 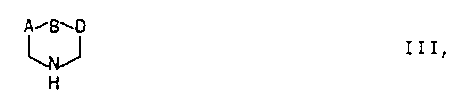
in der A, B und D die für Formel I angegebenen Bedeutungen haben, umsetzt und die so erhaltene Verbindung gegebenenfalls in das Säureadditionssalz einer physiologisch verträglichen Säure überführt.

Als nukleofuge Abgangsgruppe für Nu kommen vorzugsweise Halogenatome, insbesondere Brom oder Chlor, in Betracht.

Die Umsetzung erfolgt zweckmäßig in Gegenwart einer inerten Base, wie Triethylamin oder Kaliumcarbonat, als säurebindendes Mittel in einem inerten Lösungsmittel, wie einem cyclischen gesättigten Ether, insbesondere Tetrahydrofuran oder Dioxan, oder einem Benzolkohlenwasserstoff, wie Toluol oder Xylol.

Die Umsetzung erfolgt in der Regel bei Temperaturen von 80 bis 150°C, und ist im allgemeinen innerhalb von 1 bis 10 Stunden beendet.

Die Produkte der Formel I können durch weiterführende Reaktionen wie in den Beispielen angedeutet umgewandelt werden.

Diese Reaktionen beinhalten Oxidationen der 3-Hydroxymethylpiperidin-Struktur (R⁷ = OH in Formel I) zu den entsprechenden Carbonyl-Derivaten mit Jones-Reagens (Chrom(VI)-oxid in 25 %iger Schwefelsäure), die Reduktion der Butan-2-on-Teilstruktur (R² + R³ = Sauerstoff) zum entsprechenden Butan-2-ol-Derivat mit Natriumboranat, die H₂O-Eliminierung der 4-Hydroxy-piperidin-Teilstruktur (R⁵ = OH) zum Δ⁴-Dehydropiperidin-Derivat mit konzentrierter Schwefelsäure sowie die basenkatalysierte Doppelbindungverschiebung zur Δ³-Dehydro-piperidin-Verbindung.

Die erfindungsgemäßpen Verbindungen der Formel I werden in der Regel in Form gelblicher bis gelber Kristalle erhalten und können durch Umkristallisation aus den üblichen organischen Lösungsmitteln, bevorzugt aus einem niederen Alkohol, wie Ethanol, umkristallisiert oder durch Säulenchromatographie gereinigt werden.

Die freien 1,3,4-trisubstituierten Piperidin-Derivate der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die erfindungsgemäßen Verbindungen weisen wertvolle pharmakologische Eigenschaften auf. Sie können als Neuroleptika, Antidepressiva, Sedativa, Hypnotika oder Gehirnprotektiva Verwendung finden. Mehrere der genannten Wirkungsqualitäten können kombiniert bei einer erfindungsgemäßen Verbindung auftreten.

Sie eignen sich daher zur Behandlung von Psychosen - vorzugsweise Schizophrenie - und Unruhezuständen, zur Behandlung und Vorbeugung von Schlaganfällen oder organisch bedingten zerebralen Funktionsstörungen und zur Behandlung von Schlafstörungen.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 2 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

Die als Ausgangsstoffe für die Synthese der neuen Verbindungen benötigten Substanzen der Formel II sind bekannt.

Die Substanzen der Formel III sind bislang nicht beschrieben (siehe aber DE 41 12 352). Sie werden z.B. hergestellt, in dem man 2 Moläquivalente eines α,β-ungesättigten Ketons der Formel IV

CH₂=CH-CO-R⁴ IV

oder ein β-Halogenketon der Formel V

Hal-CH₂-CH₂-CO-R⁴ V,

worin R⁴ die oben angegebene Bedeutung besitzt, mit einem Amin H₂N-R⁸, wobei R⁸ eine gegebenenfalls durch ein Halogenatom, eine Methoxy- oder Nitrogruppe substituierter Benzylrest oder eine Allylgruppe ist, in Gegenwart von 1 Moläquivalent Natronlauge in Methanol bei 50°C umsetzt. Dabei erhält man meist diastereoselektiv die Verbindung VI mit diäquatorialer trans-Konfiguration der Substituenten R⁴ 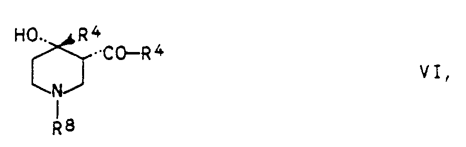
die sich durch Abspaltung von R⁸ und sich gegebenenfalls anschließende Wasserabspaltung und eventuelle Umlagerung sowie Reduktion wie oben für die Endprodukte beschrieben in die Verbindungen der Formel III überführen läßt.

Die folgenden Beispiele dienen zur Erläuterung der Erfindung:

### Beispiel 1

### a) Herstellung des Ausgangsmaterials trans-(3-Phenylhydroxymethyl-4-phenyl)-4-hydroxy-piperidin

600 g (1,62 M) trans-3-Benzoyl-4-phenyl-4-hydroxy-1-benzylpiperidin in 1,4 l Essigester gemischt mit 1,4 l Methanol wurden unter Zusatz von 20 g Palladium (10 %ig) auf Kohle 12 h bei 70°C unter einem Wasserstoffdruck von 100 bar katalytisch hydriert. Der Ansatz wurde unter Erwärmen auf 50°C vom Katalysator filtriert, zur Trockene eingeengt, in 0,6 l Aceton aufgenommen und unter Kühlen gerührt. Das ausgefallene Produkt saugte man ab, engte das Filtrat ein, rührte den Rückstand wiederum unter Kühlen in 300 ml Aceton und saugte eine 2. Fraktion an Produkt ab. Ausbeute: 345 g (75 %) des einheitlichen Produktdiastereomeren, Schmp.: 167-169°C.

### b) Herstellung des Endprodukts 1-(4-Fluor-phenyl)-4-[trans-(3-phenylhydroxymethyl-4-phenyl)-4-hydroxy-piperidin-1-yl]-butan-1-on

20,0 g (70,7 mM) trans-(3-Phenylhydroxymethyl-4-phenyl)-4-hydroxy-piperidin in 210 ml Xylol wurden mit 26,7 ml (162 mM) ω-Chlor-4-fluor-butyrophenon sowie mit 18,8 g (136 mM) fein pulverisiertem Kaliumcarbonat nebst 1,0 g Kaliumjodid versetzt und unter gutem Rühren 16 h unter Rückfluß gekocht. Nach dem Abkühlen gab man noch 200 ml Toluol hinzu und ließ die hellen Festkörper unter intensivem Rühren ausfallen. Das Rohprodukt wurde abgesaugt, mit Toluol gewaschen und im Vakuum bis 50°C getrocknet. Die Festkörper digerierte man anschließend in 1 l Wasser bei 50°C und saugte schließlich Produkt (Schmp.: 182-183°C) ab, Ausbeute: 22,6 g (72 %).

Analog lassen sich herstellen:
2. 1-Phenyl-4-[trans-(3-phenylhydroxymethyl-4-phenyl)-4-hydroxy-piperidin-1-yl]-butan-1-on,
   Schmp.: 173 bis 175°C
3. 1-(4-Brom-phenyl)-4-[trans-(3-phenylhydroxymethyl-4-phenyl)-4-hydroxy-piperidin-1-yl]-butan-1-on,
   Schmp.: 186-187°C

### Beispiel 4

### a) Herstellung des Ausgangsmaterials trans-(3-p-Fluorphenylhydroxymethyl-4-fluorphenyl)-4-hydroxy-piperidin

30,0 g (67,6 mM) trans-(3-p-Fluorbenzoyl-4-p-fluorphenyl)-4-hydroxy-1-benzyl-piperidin-Hydrochlorid in 1 l Methanol wurden unter Zusatz von 4,0 g Palladium (10 %ig) auf Kohle 8 h bei 50°C katalytisch hydriert. Nach Filtrieren und Nachwaschen mit Methanol wurde das Filtrat zur Trockene eingeengt. Den Rückstand nahm man in einer Mischung von 140 ml Methanol und 500 ml Wasser bei 70°C auf, gab bis zur alkalischen Reaktion konzentrierten Ammoniak hinzu und saugte das ausgefallene Produkt unter gutem Nachwaschen mit Wasser ab. Man isolierte nach Trocknen im Vakuum bei 60°C 20,2 g (94 %) Produkt, Schmp. 215 bis 217°C.

### b) Herstellung des Endprodukts 1-(4-Fluor-phenyl)-4-[trans-(3-p-fluorphenylhydroxymethyl-4-p-fluorphenyl)-4-hydroxypiperidin-1-yl]-butan-1-on

20,0 g (63,0 mM) trans-(3-p-Fluorphenyl-hydroxymethyl-4-p-fluorphenyl)-4-hydroxy-piperidin in einer Mischung von 250 ml Toluol und 25 ml Dimethylformamid wurden mit 20,6 ml (125 mM) ω-Chlor-4-fluor-butyrophenon sowie mit fein pulverisiertem Kaliumcarbonat nebst 1,0 g Kaliumjodid versetzt und unter gutem Rühren 8 h unter Rückfluß gekocht. Nach dem Abkühlen engte man am Rotationsverdampfer ein, nahm den Rückstand in wenig Dimethylformamid auf und goß die Lösung unter gutem Rühren auf Eiswasser. Die ausfallenden Festkörper wurden abgesaugt, gut mit Wasser nachgewaschen und aus Ethanol umkristallisiert. Man erhielt 23,5 g (78 %) Produkt, Schmp. 175 bis 177°C.

Analog lassen sich herstellen:
5. 1-(Phenyl)-4-[trans-(3-p-fluorphenylhydroxymethyl-4-p-fluorphenyl)-4-hydroxy-piperidin-1-yl]-butan-1-on

### Beispiel 6

### a) Herstellung des Ausgangsmaterials cis-(3-Carbonylmethyl-4-methyl)-4-hydroxy-piperidin

20,1 g (81,0 mM) cis-(3-Carbonylmethyl-4-methyl)-4-hydroxy-1-benzyl-piperidin in 700 ml Methanol wurden unter Zusatz von 2,5 g Palladium (10 %ig) auf Kohle 8 h bei Raumtemperatur katalytisch hydriert. Nach Filtrieren und Nachwaschen mit Methanol wurde das Filtrat zur Trockene eingeengt. Man isolierte 11,9 g (94 %) Produkt, Schmp. 91 bis 93°C. Das Hydrochlorid schmilzt bei 118 bis 199°C.

Analog läßt sich das trans-Isomere herstellen: Schmp. ab 133°C Zersetzung (Hydrochlorid).

### b) Herstellung des Endprodukts

### 1-(4-Fluorphenyl)-4-[cis-(3-carbonylmethyl-4-methyl)-4-hydroxy-piperidin-1-yl]-butan-1-on

20,5 g (131 mM) cis-(3-Carbonylmethyl-4-methyl)-4-hydroxypiperidin wurden in einer Mischung von 250 ml Toluol und 25 ml Dimethylformamid mit 21,4 ml (131 mM) ω-Chlor-4-fluorbutyrophenon sowie mit 18 g (131 mM) fein pulverisiertem Kaliumcarbonat nebst 1,0 g Kaliumjodid versetzt und unter gutem Rühren 3 h unter Rückfluß gekocht. Nach dem Abkühlen engte man am Rotationsverdampfer ein und verteilte den Rückstand zwischen Methylenchlorid und Wasser. Die wäßrige Phase wurde mit Methylenchlorid nachextrahiert und darauf die organische Phase nach dem Trocknen mit Natriumsulfat eingeengt. Das Rohprodukt reinigte man durch Säulenchromatographie (Kieselgel, Laufmittel Methylenchlorid/Methanol 98/2). Man isolierte 9,5 g (22 %) Produkt, Schmp. ab 90°C Zersetzung (Tartrat).

Analog lassen sich herstellen:
7. 1-(4-Fluorphenyl)-4-[trans-(3-carbonylmethyl-4-methyl)-4-hydroxy-piperidin-1-yl]-butan-1-on,
   Schmp. 174 bis 175°C (Hydrochlorid)
8. 1-(4-Fluorphenyl)-4-[cis-(3-carbonylmethyl-4-methyl)-4-hydroxy-piperidin-1-yl]-butan-1-ol,
   Alkylierungsreagens: 1-Hydroxy-1-(p-fluorphenyl)-4-chlorbutan
9. 1-(4-Fluorphenyl)-4-[trans-(3-carbonylmethyl-4-methyl)-4-hydroxy-piperidin-1-yl]-butan-1-ol, Schmp. 97 bis 99°C, Alkylierungsreagens: 1-Hydroxy-1-(p-fluorphenyl)-4-chlorbutan
10. 1-(4-Phenyl)-4-[cis-(3-carbonylmethyl-4-methyl)-4-hydroxypiperidin-1-yl]-butan-1-on
11. 1-(4-Phenyl)-4-[trans-(3-carbonylmethyl-4-methyl)-4-hydroxypiperidin-1-yl]-butan-1-on
12. 1-(4-Fluorphenyl)-4-[trans-(3-carbonylethyl-4-ethyl)-4-hydroxy-piperidin-1-yl]-butan-1-on

### Beispiel 13

### 1-(4-Fluor-phenyl)-4-[trans-(3-phenyl-hydroxymethyl-4-phenyl)-4-hydroxy-piperidin-1-yl]-butan-1-ol

5,0 g (11,6 mM) 1-(4-Fluor-phenyl)-4-[trans-(3-phenylhydroxymethyl-4-phenyl)-4-hydroxy-piperidin-1-yl]-butan-1-on (Beispiel 1b) wurden in einer Mischung aus 80 ml Methanol und 100 ml Tetrahydrofuran gelöst und mit 0,6 g (16 mM) Natriumboranat versetzt. Man ließ noch 2 h bei Raumtemperatur nachrühren und engte am Rotationsverdampfer ein. Den Kolbenrückstand verteilte man zwischen Methylenchlorid und Wasser bei pH = 10 und engte die organische Phase nach dem Trocknen mit Natriumsulfat ein. Man isolierte 4,6 g (88 %) Produkt, Schmp.: 90 bis 92°C (Zersetzung).

Analog lassen sich herstellen:
14. 1-Phenyl-4-[trans-(3-phenylhydroxymethyl-4-phenyl)-4-hydroxy-piperidin-1-yl]-butan-1-ol
   Schmp.: 90 bis 92°C
15. 1-(4-Fluor-phenyl)-4-[trans-(3-p-fluorphenylhydroxymethyl-4-p-fluorphenyl)-4-hydroxy-piperidin-1-yl]-butan-1-ol
16. 1-Phenyl-4-[trans-(3-p-fluorphenyl-hydroxymethyl-4-p-fluorphenyl)-4-hydroxy-piperidin-1-yl]-butan-1-ol
17. 1-(4-Fluor-phenyl)-4-[cis-(3-α-hydroxyethyl-4-methyl)-4-hydroxy-piperidin-1-yl]-butan-1-ol
18. 1-(4-Fluor-phenyl)-4-[trans-(3-α-hydroxyethyl-4-methyl)-4-hydroxy-piperidin-1-yl]-butan-1-ol
19. 1-(4-Brom-phenyl)-4-[trans-(3-phenylhydroxymethyl-4-phenyl)-4-hydroxypiperidin-1-yl]-butan-1-ol,
   Schmp.: 92 bis 93°C

### Beispiel 20

### 1-(4-Fluor-phenyl)-4-[trans-(3-benzoyl-4-phenyl)-4-hydroxypiperidin-1-yl]-butan-1-on

22,6 g (50,7 mM) 1-(4-Fluor-phenyl)-4-[trans-(3-(phenylhydroxymethyl-4-phenyl)-4-hydroxy-piperidin-1-yl]-butan-1-on (Beispiel 1b) suspendierte man in 500 ml Aceton und tropfte anschließend unter gutem Rühren 21 ml (56 mM) Jones-Reagenz hinzu. Dabei stieg die Temperatur auf 32°C. Man ließ noch 12 h bei Raumtemperatur nachrühren und engte dann am Rotationsverdampfer weitgehend ein. Anschließend gab man den Ansatz auf Eiswasser, unterschichtete mit Methylenchlorid, machte mit verdünnter Natronlauge alkalisch und saugte den ausgefallenen Chromoxid-Niederschlag ab. Die wäßrige Phase wurde mit Methylen-chlorid nachextrahiert und darauf die organische Phase nach dem Trocknen mit Natriumsulfat eingeengt. Man isolierte 21,1 g (95 %) Produkt, Schmp.: 128 bis 130°C.

Analog lassen sich herstellen:
21. 1-(4-Fluor-phenyl)-4-[trans-(3-p-fluorbenzoyl-4-p-fluorphenyl)-4-hydroxy-piperidin-1-yl]-butan-1-on,
   Schmp.: 111 bis 113°C
22. 1-Phenyl-4-[trans-(3-p-fluorbenzoyl-4-p-fluorphenyl)-4-hydroxypiperidin-1-yl]-butan-1-on

### Beispiel 23

### a) Herstellung des Ausgangsmaterials

### 1. trans-(3-Benzoyl-4-phenyl)-4-hydroxy-piperidin

Zu 6,0 g (21,2 mM) trans-(3-Phenylhydroxymethyl-4-phenyl)-4-hydroxy-piperidin (Beispiel 1a) in 150 ml Aceton wurden 8 ml (21,4 mM) Jones-Reagenz zugetropft. Dabei stieg die Temperatur bis auf 30°C. Man ließ noch 1 h bei Raumtemperatur nachrühren, dekantierte vom ausgefallenen Chromoxid-Niederschlag ab und engte am Rotationsverdampfer auf die Hälfte ein. Anschließend gab man den Ansatz auf Eiswasser machte mit verdünnter Natronlauge alkalisch, extrahierte mehrmals mit Methylenchlorid und engte die organische Phase nach dem Trocknen mit Natriumsulfat ein. Man isolierte 5,2 g (87 %) Produkt, das nach Umkristallisieren aus Essigester bei 128-129°C schmolz.

### 2. 3-Benzoyl-4-phenyl-Δ⁴-dehydro-piperidin

Zu 186 g (1,9 M) konzentrierter Schwefelsäure tropfte man unter Eiskühlung 400 ml Methylenchlorid und anschließend 112,6 g (304 mM) trans-3-Benzoyl-4-phenyl-4-hydroxy-piperidin gelöst in 350 ml Methylenchlorid bei einer Innentemperatur von 0 bis 5°C hinzu. Man ließ 2 bis 3 h bei Eiskühlung rühren, gab dann den Ansatz auf Eiswasser, gab konzentrierter Natronlauge hinzu (bis pH 10), verteilte zwischen Methylenchlorid und Wasser und engte die organische Phase nach dem Trocknen mit Natriumsulfat ein. Das Rohprodukt reinigte man durch Säulenchromatographie (Kieselgel, Laufmittel Methylenchlorid + 5 % Methanol), Ausbeute: 61 g (57 %), Schmp.: 118 bis 119°C.

### b) Herstellung des Endprodukts

### 1-(4-Fluor-phenyl)-4-[3-benzoyl-4-phenyl-Δ⁴-dehydro-piperidin-1-yl]-butan-1-on

15,4 g (59 mM) 3-Benzoyl-4-phenyl-Δ⁴-dehydro-piperidin in 90 ml Xylol wurden mit 11,5 ml (70 mM) ω-Chlor-4-fluor-butyrophenon sowie mit 12,1 g (88mM) fein pulverisiertem Kaliumcarbonat nebst 0,5 g Kaliumjodid versetzt und unter gutem Rühren 15 h am Rückfluß gekocht. Man engte den Ansatz am Rotationsverdampfer weitgehend ein. Anschließend verteilte man den Rückstand zwischen Eiswasser und Methylenchlorid und machte mit verdünnter Natronlauge alkalisch. Die wäßrige Phase wurde mit Methylenchlorid nachextrahiert und darauf die organische Phase nach dem Trocknen mit Natriumsulfat eingeengt. Das Rohprodukt reinigte man durch Säulenchromatographie (Kieselgel, Laufmittel Methylenchlorid/Methanol 98/2). Man isolierte 5,2 g (21 %) Produkt, Schmp.: 89-91°C (Hydrochlorid).

Analog läßt sich herstellen:
24. 1-(4-Fluor-phenyl)-4-[3-benzoyl-4-phenyl-Δ⁴-dehydropiperidin-1-yl]-butan-1-ol

### Beispiel 25

### 1-(4-Fluor-phenyl)-4-[3-benzoyl-4-phenyl-Δ⁴-dehydro-piperidin-1-yl]-butan-1-on

Zu 18,0 ml (336 mM) konzentrierter Schwefelsäure tropfte man unter Eiskühlung 100 ml Methylenchlorid und anschließend 10,0 g (22,4 mM) 1-(4-Fluor-phenyl)-4-[trans-(3-benzoyl-4-phenyl)-4-hydroxy-piperidin-1-yl]-butan-1-on (Beispiel 20) gelöst in 50 ml Methylenchlorid bei einer Innentemperatur von 0 bis 5°C. Man ließ 2 h bei Eiskühlung nachrühren, gab dann den Ansatz auf Eiswasser, stellte mit konzentrierter Natronlauge auf pH 10, verteilte zwischen Methylenchlorid und Wasser und engte die organische Phase nach dem Trocknen mit Natriumsulfat ein. Das Rohprodukt reinigte man durch Säulenchromatographie (Kieselgel, Laufmittel Methylenchlorid 99/1), Ausbeute: 4,2 g (44 %) helles öl. Das Hydrochlorid schmilzt bei 89 bis 91°C.

Analog lassen sich herstellen:
26. 1-(4-Fluor-phenyl)-4-[3-p-fluorbenzoyl-4-fluorphenyl-Δ⁴-dehydro-piperidin-1-yl]-butan-1-on
27. 1-Phenyl-4-[3-benzoyl-4-phenyl-Δ⁴-dehydropiperidin-1-yl]-butan-1-on
28. 1-phenyl-4-[3-p-fluorbenzoyl-4-p-fluorphenyl-Δ⁴-dehydropiperidin-1-yl]-butan-1-on

### Beispiel 29

### 1-(4-Fluor-phenyl)-4-[3-carbonylmethyl-4-methyl-Δ³-deyhdropiperidin-1-yl]-butan-1-on

Zu 40 ml (747 mM) konzentrierter Schwefelsäure tropfte man unter Eiskühlung 200 ml Methylenchlorid und anschließend 22,0 g (68,5 mM) 1-(4-Fluor-phenyl)-4-[cis,trans-(3-carbonylmethyl-4-methyl)-4-hydroxy-piperidin-1-yl]-butan-1-on (Beispiel 6, 7) gelöst in 100 ml Methylenchlorid bei einer Innentemperatur von 0 bis 5°C. Man ließ 2 h bei Eiskühlung und anschließend 1 h unter Erwärmen auf 30 bis 35°C nachrühren, gab dann den Ansatz auf Eiswasser, stellte mit konzentrierter Natronlauge auf pH 10, verteilte zwischen Methylenchlorid und Wasser und engte die organische Phase nach dem Trocknen mit Natriumsulfat ein. Das Rohprodukt reinigte man durch Säulenchromatographie (Kieselgel, Laufmittel Methylenchlorid 99/1), Ausbeute: 9,8 g (45 %), Schmp. 67 bis 70°C (Tartrat).

Analog lassen sich herstellen:
30. 1-Phenyl-4-[3-carbonylmethyl-4-methyl-Δ³-deyhdro-piperidin-1-yl]-butan-1-on

### Beispiel 31

### 1-(4-Fluor-phenyl)-4-[3-(phenyl)-hydroxymethyl-4-phenyl-Δ⁴-dehydro-piperidin-1-yl]-butan-1-on

4,0 g (9,0 mM) 1-(4-Fluor-phenyl)-4-[3-benzoyl-4-phenyl-Δ⁴-dehydro-piperidin-1-yl]-butan-1-on (Beispiel 23, 25) wurde in 50 ml Methanol suspendiert und langsam bei 30°C mit 0,34 g (9,0 mM) Natriumboranat versetzt. Man ließ noch 2 h bei Raumtemperatur nachrühren und engte am Rotationsverdampfer ein. Den Kolbenrückstand verteilte man zwischen Methylenchlorid und Wasser bei pH = 10 und engte die organische Phase nach dem Trocknen mit Natriumsulfat ein. Man isolierte 3,8 g (95 %) Produkt,
Schmp.: 191 bis 192°C (Hydrochlorid).

### Beispiel 32

### a) Herstellung des Ausgangsmaterials

### 3-Benzoyl-4-phenyl-Δ³-dehydro-piperidin

Zu 4,2 g (16,0 mM) 3-Benzoyl-4-phenyl-Δ⁴-dehydro-piperidin (Beispiel 23 a), in 60 ml Methanol fügte man 12,0 g (66 mM) 30 % Natriummethylat Lösung hinzu und ließ das Reaktionsgemisch 8 h unter Rückfluß kochen. Nach Rühren über Nacht bei Raumtemperatur engte man den Ansatz am Rotationsverdampfer vollständig ein. Den Kolbenrückstand gab man auf Eiswasser, extrahierte mehrmals Methylenchlorid und engte die organischen Phasen nach dem Trocknen mit Natriumsulfat ein. Das Rohprodukt reinigte man durch Säulenchromatographie (Kieselgel, Laufmittel Methylenchlorid + 1 % Methanol), Ausbeute: 1,9 g (45 %), Schmp.: 189 bis 192°C.

### b) Herstellung des Endprodukts

### 1-(4-Fluor-phenyl)-4-[3-benzoyl-4-phenyl-Δ³-dehydro-piperidin-1-yl]-butan-1-on

2,8 g (10,6 mM) 3-Benzoyl-4-phenyl-Δ³-dehydro-piperidin in 50 ml Xylol wurde mit 2,6 ml (15,4 mM) ω-Chlor-4-fluor-butyrophenon sowie mit 2,2 g (16 mM) fein pulverisiertem Kaliumcarbonat nebst 0,5 g Kaliumjodid versetzt und unter gutem Rühren 13 h am Rückfluß gekocht. Den Ansatz verteilte man zwischen Methylenchlorid und Wasser und engte die organische Phase nach dem Trocknen mit Natriumsulfat ein. Das Rohprodukt reinigte man durch Säulenchromatographie (Kieselgel, Laufmittel Methylenchlorid + 5 % Methanol), Ausbeute: 1,4 g (31 %) Produkt mit Schmp. 171 bis 172°C.

Analog läßt sich herstellen:
33. 1-(4-Fluor-phenyl)-4-[3-benzoyl-4-phenyl-Δ³-dehydro-piperidin-1-yl)-butan-1-ol

### Beispiel 34

### 1-(4-Fluor-phenyl)-4-[3-benzoyl-4-phenyl-Δ³-dehydro-piperidin-1-yl]-butan-1-on

Zu 4,5 g (10,5 mM) 1-(4-Fluor-phenyl)-4-[3-benzoyl-4-phenyl-Δ⁴-dehydro-piperidin-1-yl]-butan-1-on (Beispiel 23, 25) in 60 ml Methanol fügte man 5,7 g (32 mM) 30 % Natriummethylatlösung hinzu und ließ das Reaktionsgemisch 1,5 h unter Rückfluß kochen. Nach Rühren über Nacht bei Raumtemperatur engte man den Ansatz am Rotationsverdampfer vollständig ein. Den Kolbenrückstand gab man in Eiswasser, verteilte zwischen Methylenchlorid und Wasser, stellte auf pH = 10 ein und engte die organische Phase nach dem Trocknen mit Natriumsulfat ein. Das Rohprodukt reinigte man durch Säulenchromatographie (Kieselgel, Laufmittel Methylenchlorid + 2,5 % Methanol), Ausbeute: 2,2 g (48 %), das Maleinsäuresalz schmilzt bei 171 bis 172°C.

Analog lassen sich herstellen:
35. 1-(4-Fluor-phenyl)-4-[3-p-fluorbenzoyl-4-p-fluorphenyl-Δ³-dehydro-piperidin-1-yl]-butan-1-on, Schmp. 211 bis 213°C (Hydrochlorid)
36. 1-Phenyl-4-[3-benzoyl-4-phenyl-Δ³-dehydro-piperidin-1-yl]-butan-1-on
37. 1-Phenyl-4-[3-p-fluorbenzoyl-4-p-fluorphenyl-Δ³-dehydro-piperidin-1-yl]-butan -1-on

### Beispiel 38

### a) Herstellung des Ausgangsmaterials cis-(3-Phenylhydroxymethyl-4-phenyl)-piperidin

7,8 g (22,1 mM) 3-Benzoyl-4-phenl-1-benzyl-Δ⁴-dehydro-piperidin (hergestellt analog Beispiel 23a) in 300 ml Ethanol wurden unter Zusatz von 1,6 g Palladium (10 %ig) auf Kohle 48 h bei Normaldruck und Raumtemperatur katalytisch hydriert. Der Ansatz wurde vom Katalysator filtriert, zur Trockene eingeengt, unter Erwärmen in 40 ml Aceton aufgenommen und unter Kühlen gerührt. Das ausgefallene Produkt saugte man ab und wusch mit Aceton nach. Ausbeute: 2,3 g (39 %), das Hydrochlorid schmilzt bei 239 bis 240°C.

### b) Herstellung des Endprodukts 1-(4-Fluor-phenyl)-4-[cis-(3-phenylhydroxymethyl-4-phenyl)-piperidin-1-yl]-butan-1-on

4,0 g (15,0 mM) cis-(3-Phenylhydroxymethyl-4-phenyl)-piperidin in 50 ml Toluol wurde mit 3,8 ml (23 mM) ω-Chlor-4-fluor-butyrophenon sowie mit fein pulverisiertem Kaliumcarbonat nebst 1,0 g Kaliumjodid versetzt und unter gutem Rühren 25 h unter Rückfluß gekocht. Nach dem Abkühlen engte man das Filtrat ein, verteilte den Rückstand bei pH 10 zwischen Methylenchlorid und Wasser und engte die organische Phase nach dem Trocknen ein. Das Rohprodukt reinigte man durch Säulenchromatographie (Kieselgel, Laufmittel Methylenchlorid + 5 % Methanol). Man isolierte 3,5 g (54 %) Produkt, Schmp. 113 bis 114°C.

Analog lassen sich herstellen:
39. 1-(4-Fluor-phenyl)-4-[cis-(3-p-fluorphenyl-hydroxymethyl-4-p-fluorphenyl)-piperidin-1-yl]-butan-1-on
40. 1-(4-Fluor-phenyl)-4-[cis-3-(carbonylmethyl-4-methyl)-piperidin-1-yl]-butan-1-on

## Patentansprüche

1. 1,3,4-trisubstituierte Piperidin-Derivate der Formel I 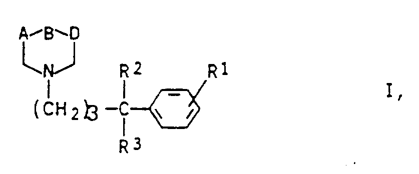 worin
R¹ ein Wasserstoff-, Fluor-, Chlor- oder Bromatom bedeutet,
R² einen Hydroxyrest, einen gegebenenfalls durch ein Fluor-, Chlor- oder Bromatom substituierten Phenylrest darstellt,
R³ ein Wasserstoffatom bedeutet oder
R² und R³ zusammen ein Sauerstoffatom darstellen, und
A-B-D die Gruppen 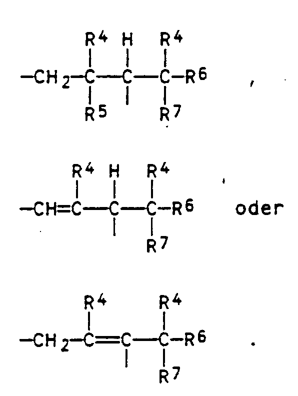 darstellt, worin
R⁴ einen C₁₋₃-Alkylrest oder eine Phenyl- oder Thienylgruppe darstellt, die durch ein Fluor- oder Chloratom substituiert sein können,
R⁵ ein Wasserstoffatom oder eine Hydroxygruppe darstellt,
R⁶ ein Wasserstoffatom darstellt,
R⁷ eine Hydroxygruppe ist oder
R⁶ und R⁷ zusammen ein Sauerstoffatom bedeuten
und ihre Salze mit physiologisch verträglichen Säuren.

2. Verfahren zur Herstellung der Verbindungen der Formel I gemäß Anspruch 1, dadurch gekennzeichnet, daß man eine Verbindung der Formel II 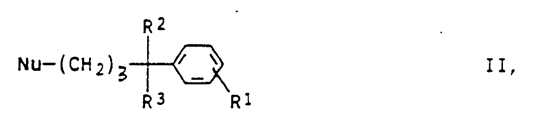 in der R¹, R² und R³ die angegebenen Bedeutungen haben und Nu eine nukleofuge Abgangsgruppe darstellt, mit einem 3,4-disubstituierten Piperidin-Derivat der Formel III 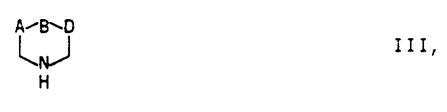 in der A, B und D die für Formel I angegebenen Bedeutungen haben, umsetzt und die so erhaltene Verbindung gegebenenfalls in das Säureadditionssalz einer physiologisch verträglichen Säure überführt.

3. Verbindungen der Formel I gemäß Anspruch zur Verwendung bei der Bekämpfung von Krankheiten.

## Claims

1. A 1,3,4-trisubstituted piperidine derivative of the formula I 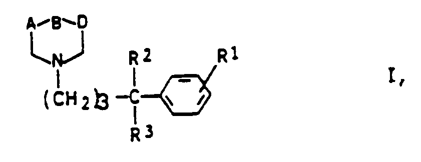 where
R¹ is hydrogen, fluorine, chlorine or bromine,
R² is hydroxyl, or phenyl which is unsubstituted or substituted by fluorine, chlorine or bromine,
R³ is hydrogen, or
R² and R³ together are oxygen, and
A-B-D is 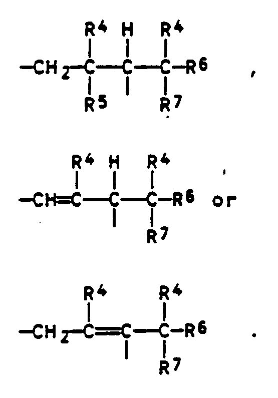 where
R⁴ is C₁₋₃-alkyl or is phenyl or thienyl which can be substituted by fluorine or chlorine,
R⁵ is hydrogen or hydroxyl,
R⁶ is hydrogen,
R⁷ is hydroxyl, or
R⁶ and R⁷ together are oxygen,
and its salts with physiologically tolerated acids.

2. A process for preparing a compound of the formula I as claimed in claim 1, which comprises reacting a compound of the formula II 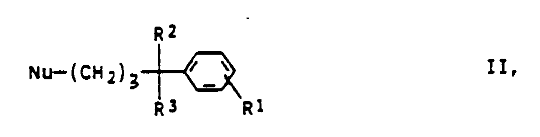 where R¹, R² and R³ have the stated meanings, and Nu is a nucleofugic leaving group, with a 3,4-disubstituted piperidine derivative of the formula III 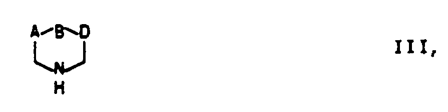 where A, B and D have the meanings stated for formula I, and converting the resulting compound where appropriate into the addition salt with a physiologically tolerated acid.

3. A compound of the formula I as claimed in claim 1 for use for controlling diseases.

## Revendications

1. Dérivés de pipéridine trisubstitués sur 1,3,4 de la formule I 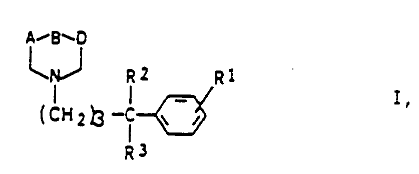 dans laquelle
R¹ représente un atome d'hydrogène, de fluor, de chlore ou de brome,
R² représente un reste hydroxy, un reste phényle éventuellement substitué par un atome de fluor, de chlore ou de brome,
R³ représente un atome d'hydrogène ou
R² et R³ ensemble représentent un atome d'oxygène, et
A-B-D représente les groupes 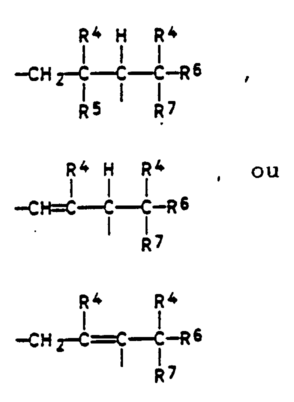 R⁴ représente un reste alkyle en C1-C3 ou un groupe phényle ou thiényle qui peuvent être substitués par un atome de fluor ou de chlore,
R⁵ représente un groupe hydroxy ou un atome d'hydrogène,
R⁶ représente un atome d'hydrogène,
R⁷ est un groupe hydroxy ou
R⁶ et R⁷ ensemble représentent un atome d'oxygène et leurs sels d'acides acceptables physiologiquement.

2. Procédé de préparation des composés de formule I selon la revendication 1, caractérisé par le fait qu'on fait réagir un composé de la formule II 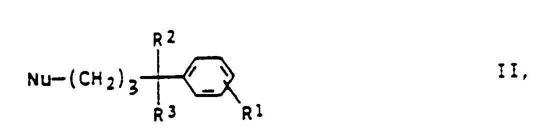 dans laquelle R¹, R² et R³ ont les significations indiquées et Nu représente un groupe séparable nucléofuge, avec un dérivé de pipéridine disubstitué sur 3,4 de la formule III 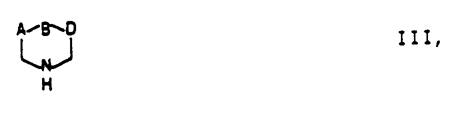 dans laquelle A, B et D ont les significations indiquées pour la formule I, et on transforme le composé obtenu, éventuellement en le sel d'addition d'acide d'un acide acceptable plysiologiquement.

3. Composés de la formule I selon la revendication 1 pour utilisation pour la lutte contre les maladies.
